(19) Europäisches Patentamt
European Patent Office
Office européen des brevets

(11) **EP 3 417 771 A1**

(12) **EUROPEAN PATENT APPLICATION**

(43) Date of publication:
26.12.2018 Bulletin 2018/52

(51) Int Cl.:
*A61B 5/021* (2006.01)

(21) Application number: 18178332.5

(22) Date of filing: 18.06.2018

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 20.06.2017 US 201715628422

(71) Applicant: **Heartisans Limited**
**Hong Kong (HK)**

(72) Inventor: **LUI, Hin Wai**
**Wan Chai (HK)**

(74) Representative: **Abraham, Richard et al**
**Maguire Boss**
**24 East Street**
**St. Ives, Cambridgeshire PE27 5PD (GB)**

(54) **A METHOD FOR MONITORING BLOOD PRESSURE, AND A DEVICE THEREOF**

(57) A device and a method which monitors the pulse transit time of a heart beat is disclosed, i.e. the time taken for a heart impulse to travel from the heart to a limb of a subject. The time taken to travel is calibrated to blood pressure of the subject by a pre-determined formula. However, the calibration is augmented by empirical observations, obtaining several optional calibrations. The most suitable of the calibrations is selected to interpret each pulse transit time reading based on proximity of the pulse transit time. Hence, it is possible that every pulse transit time reading may use a different calibration. The method diminishes the effect of physiological change in the subject which causes drift in a solely formula based calibration.

Figure 3

**Description**

**BACKGROUND**

The current invention relates to devices and methods for measuring blood pressure.

High blood pressure and low blood pressure are symptoms which are associated with different illnesses. While having a hypertension or a low blood pressure is itself not a disease, and hence is not a diagnosis of any disease, monitoring blood pressure aids in identifying the underlying diseases. For example, high blood pressure may provide warning of possible heart attack or stroke, having high blood pressure itself is not having a heart attack or stroke.

Traditionally, a home based blood pressure monitor comprises a sphygmomanometer with an inflatable cuff which can strangle the subject's bicep to provide a referential pressure. Use of such a blood pressure monitor creates considerable discomfort, and may even cause pain to users. The setup and measurement procedure using such an inflatable cuff blood pressure monitor is often time-consuming and inconvenient, requiring the subject to insert his arm through the cuff while operating the blood pressure monitor with the other hand, and often necessitate the help of family members. The size and the bulk of such an inflatable cuff blood pressure monitor is such that it cannot easily be carried around by users to locations outside of their homes, and therefore lacks true portability.

A blood pressure measurement has been proposed, which is based on measuring the pulse transit time of a heartbeat travelling from the heart to the wrist. The method does not require a cuff around the arm of a subject. However, the method has been found too inaccurate for practical use.

It is therefore desirable to provide an improved device and method for measuring blood pressure using pulse transit time of heartbeats, and which does not require an inflatable cuff, but which is sufficiently accurate for use in domestic settings. Preferably, the improved device and method is sufficiently accurate for long periods of continual monitoring.

**SUMMARY**

In a first aspect, the invention proposes a method of relating pulse transit time to blood pressure comprising the steps of: reading the pulse transit time of a current heart beat travelling from the heart to a peripheral of a subject; selecting an empirical calibration out of a plurality of empirical calibrations for interpreting the pulse transit time into blood pressure; each of the plurality of empirical calibrations having been obtained by the earlier steps of averaging a plurality of observed blood pressure and respectively observed pulse transit times to obtain an averaged observed pulse transit time and an averaged observed blood pressure, and applying the averaged observed pulse transit time and averaged observed blood pressure to either or both of formulae (1) or (2):

$$SBP = MBP_0 + \frac{2}{3} \cdot PP_0 \cdot \left(\frac{PTT_0}{PTT}\right)^2 \qquad \text{--------(1)}$$

$$DBP = MBP_0 - \frac{1}{3} \cdot PP_0 \cdot \left(\frac{PTT_0}{PTT}\right)^2 \qquad \text{--------(2)}$$

SBP = Systolic Blood Pressure
DBP = Diastolic Blood Pressure
MBP=Mean Blood Pressure
PP=Pulse Pressure
PTT=Pulse Transit Time
Subscript 0 denotes calibration point;

wherein;
the empirical calibration is selected out of the plurality of empirical calibrations based on the closest of the observed pulse transit times making up the several predetermined calibrations to the pulse transit time of the current heart beat.

In contrast to the invention, prior art blood pressure measurement which monitors pulse transit time of heartbeats does not provide the possibility to account for physiological variations of the subject's body, i.e. the body of the person whose blood pressure is monitored. However, a subject's pulse transit time can be influenced by variation in the level of activity of the sympathetic nervous system, rate of his heart beat, how the blood pressure monitoring device is worn by the subject and so on. As a result, the relationship between pulse transit time and blood pressure tends to drift naturally, despite the most careful calibration. Unfortunately, it is virtually impossible to devise a formula that takes into consideration all possible environment factors, physiological factors and all other influential factors. The invention addresses such drifts in calibration by first providing a theoretical calibration, i.e. a calibration which is based on a known or pre-determined formula. The formula is deemed sufficiently dependable to model the general relationship between pulse transit time and blood pressure. However, the invention further observes actual variations to this theoretical calibration in order to provide a plurality of empirical calibrations, i.e. calibrations based on empirical observations. Empirical calibrations vary naturally from the theoretical calibration. The best-fit of these empirical calibrations is used to determine the relationship of pulse transit time to blood pressure more accurately than theoretical calibration.

By modifying a formula-based calibration with actual variations of the formula-based calibration to provide a plurality of empirical observation-based calibrations, i.e. empirical calibrations, the invention provides the advantage of not having to identify specific physiological factors causing calibration drifts and inaccuracy. Selecting the best fit of a plurality of empirical calibrations is sufficient to address any unknown factors which contribute to drift of the calibration, especially if the empirical calibrations have been taken over a sufficiently wide range of different physiological conditions of the subject.

Typically, the theoretical calibration based upon which the empirical calibrations are obtained is a one point calibration. As calibration drift is addressed by empirical calibrations taken over a sufficiently wide range of different physiological conditions of the subject, use of multiple point calibrations is not necessary to improve accuracy.

The 'peripheral' is any distal part of the subject away from the heart, and may be his head, neck, ear and limbs. Most conveniently, however, the peripheral is the wrist of subject as a device embodying the invention may be worn on the wrist like a watch for continual, long periods of blood pressure monitoring.

Preferably, the method further comprising the steps of observing the inhalation and exhalation of the subject, i.e. the state of the subject's breath, when each pulse transit time is measured; wherein the empirical calibrations are further characterised or categorised by the state of the subject's breath; and the empirical calibration to be used to interpret any current pulse transit time is selected out of the several empirical calibrations, based on the closest of the observed pulse transit times making up the several empirical calibrations and also based on the same state of breath.

As the pulse transit time is affected by breathing, discriminating one empirical calibration from another based on the state of the breath provides an added advantage that a more accurate empirical calibration may be selected and used.

Preferably, the method further comprises the steps of: observing the period between a peak of the pulse to the same peak of the pulse immediately preceding the present pulse to obtain a peak to peak period; wherein the empirical calibrations are further characterized or categorized by peak to peak periods; and the empirical calibration is selected out of the several empirical calibrations, based on the closest of the observed pulse transit times of the several empirical calibrations and based on a similar peak to peak period.

For example, there may be one calibration for a particular pulse transit time period and for a peak to peak period of two pulses at 0.80 to 0.89 seconds, and another calibration for the same particular pulse transit time period but at a peak to peak period of 0.70 to 0.79 seconds. This discrimination between different calibrations based on the peak to peak period between any two consecutive heart beats provides the possibility of an even finer selection of the more suitable empirical calibration to interpret the current pulse transit time into blood pressure.

In other words, as the pulse transit time is affected by the period between any two consecutive heart beats, which is related to the heart rate, discriminating one calibration from another based on the peak to peak period of any two heart beats allows the best of the empirical calibrations to be selected and used.

In a second aspect, the method comprises a method of relating pulse transit time to blood pressure comprising the steps of: obtaining the pulse transit time of a current heart beat travelling from the heart to a peripheral of a subject; selecting an empirical calibration out of a plurality of empirical calibrations for interpreting the pulse transit time into blood pressure; wherein the empirical calibration is selected if the pulse transit time used to provide the empirical calibration is the nearest of all the pulse transit times used to provide the plurality of empirical calibrations to the pulse transit time of the current heart beat.

The second aspect provides the possibility that the formula in the first aspect, and the earlier steps of: averaging a plurality of observed blood pressure and respectively observed pulse transit times may be omitted, for a broader application. In a third aspect, the invention proposes a blood pressure monitor for monitoring the blood pressure of a subject, comprising an electrocardiogram sensor and a photoplethysmocharty sensor configured for observing pulse transit time of a beat from the heart to a peripheral of the subject; the blood pressure monitor configured to apply one of a plurality of calibrations to interpret pulse transit time into blood pressure; wherein the plurality of calibrations are variations of the same formula based calibration, and the variations having been observed in same subject.

Preferably but not necessarily, the formula is either or both of formulae (1) and (2):

$$SBP = MBP_0 + \frac{2}{3} \cdot PP_0 \cdot \left(\frac{PTT_0}{PTT}\right)^2 \qquad \text{-------(1)}$$

$$DBP = MBP_0 - \frac{1}{3} \cdot PP_0 \cdot \left(\frac{PTT_0}{PTT}\right)^2 \qquad \text{-------(2)}$$

SBP = Systolic Blood Pressure
DBP = Diastolic Blood Pressure
MBP=Mean Blood Pressure
PP=Pulse Pressure
PTT=Pulse Transit Time
Subscript 0 denotes calibration point;

Typically, the variations have been observed from a plurality of observed blood pressure and respectively observed pulse transit times in the same subject, and the plurality of calibrations providing the variations are groups of averaged plurality of observed blood pressure and respectively observed pulse transit times in the same subject.

Typically, the plurality of observed blood pressure and respectively observed pulse transit times are each grouped by being within 8 mm Hg of pressure difference from each other.

**.BRIEF DESCRIPTION OF DRAWINGS**

It will be convenient to further describe the present invention with respect to the accompanying drawings that illustrate possible arrangements of the invention, in which like integers refer to like parts. Other embodiments of the invention are possible, and consequently the particularity of the accompanying drawings is not to be understood as superseding the generality of the preceding description of the invention.

Figure 1 shows an embodiment of the invention;
Figure 2 shows the embodiment of the invention in the opposite side;
Figure 3 is a schematic of the components of the embodiment of Figure 1;
Figure 4 illustrates how the embodiment of Figure 1 is worn by a subject;
Figure 5 illustrates the embodiment of Figure 1 is in wireless communication with a server and a smart phone;
Figure 6 illustrates a peak to peak period of electrocardiogram pulses;
Figure 7 illustrates a peak to peak period of photoplethysmocharty pulses.
Figure 8 illustrates a pulse transit time (PTT) used in the embodiment of Figure 1;
Figure 9 illustrates a calibration method of the prior art for systolic blood pressure;
Figure 10 illustrates the calibration method of the embodiment of Figure 1 for systolic blood pressure;
Figure 11 shows the first step in providing the calibration of Figure 10;
Figure 12 shows a subsequent step in providing the calibration of Figure 10;
Figure 13 shows another subsequent step in providing the calibration of Figure 10;
Figure 14 shows another subsequent step in providing the calibration of Figure 10;
Figure 15 shows how the calibration of Figure 10 is used;
Figure 16 shows how the calibration of Figure 10 is used;
Figure 17 shows how the calibration of Figure 10 is used;
Figure 18 shows how the calibration of Figure 10 is used;
Figure 19 illustrates the calibration method of the embodiment of Figure 1 for diastolic blood pressure;
Figure 20 shows the first step in providing the calibration of Figure 19;
Figure 21 shows a subsequent step in providing the calibration of Figure 19;
Figure 22 shows another subsequent step in providing the calibration of Figure 19;
Figure 23 shows how the calibration of Figure 19 is used;
Figure 24 is a comparative example showing the accuracy of the calibration of the prior art;
Figure 25 shows the accuracy of the calibration of Figure 10 and Figure 19; and
Figure 26 illustrates how the embodiment of Figure 1 is able to monitor the inhalation and exhalation of the subject.

**DETAILED DESCRIPTION OF EMBODIMENTS**

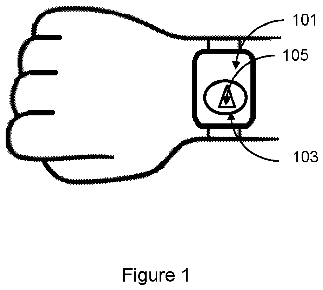

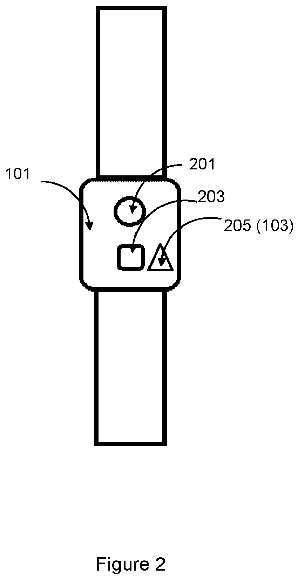

Figure 1 shows a wrist wearable blood pressure monitor 101 strapped to the wrist of a subject, i.e. the person or animal whose blood pressure is to be monitored or evaluated. Figure 2 provides a view of the underside of the blood pressure monitor 101.

On the underside of the blood pressure monitor 101 is a PPG sensor (photoplethysmocharty) sensor. A PPG sensor uses light-based technology to sense the rate of blood flow as controlled by the heart's pumping action.

In simplified description, a PPG sensor comprises at least one light source 201 such as an LED (light emitting diode) and one corresponding optical sensor 203. In use, the light source 201 transmits light onto the subject's skin, and the light is diffused and reflected by the surface of the skin and detected by the optical sensor 203. 'Reflection' is taken here to include the case wherein light penetrates beneath the skin surface but are diffused or rebounded back by the top layers of skin and tissue towards the optical sensor 203. The reflected or rebounded light will have a varying intensity which fluctuates in accordance to the pulsation of blood flow in the subject's skin and tissue. In this way, the optical sensor 203 is able to detect the heart beat of the subject. In use, the PPG sensor is capable of sampling the subject's pulse and heart beat in real time.

The blood pressure monitor 101 is designed to be worn such that the light source 201 and the optical sensor 203 are placed somewhat snugly against the skin, in order to prevent ambient light from causing too much noise signals in the optical sensor 203.

The underside of the blood pressure monitor 101 further comprises an electrode 205 of an ECG (electrocardiogram) sensor 103. On the topside of the blood pressure monitor 101 is a corresponding electrode 105 of the same ECG sensor 103. When the blood pressure monitor 101 is worn on the wrist of the subject, the electrode 205 on the underside of the blood pressure monitor 101 contacts the skin of the subject's wrist. When the subject places a finger of his other hand onto the electrode 105 on the top side of the blood pressure monitor 101, a closed electrical circuit across his heart is formed through both upper limbs. In this way, the ECG sensor 103 is able to monitor the subject's heart beat. This technology is known and does not require further elaboration here.

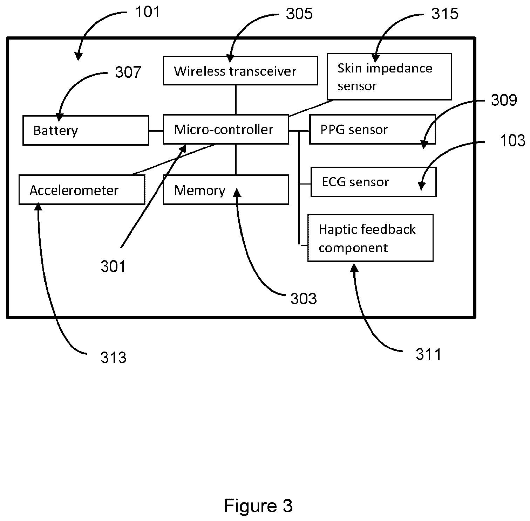

Figure 3 is a schematic diagram of one possible internal structure of the blood pressure monitor 101. The blood pressure monitor 101 comprises a microcontroller 301 and a memory 303, which are required for operation. Typically, the memory 303 contains an algorithm for analysing the pulse of the subject.

The microcontroller 301 operates the PPG sensor 309 to detect light reflected from the subject's skin, and also operates the ECG sensor 103 to detect the ECG of the subject.

A wireless transceiver 305 is also provided for wireless communication with a mobile phone or any other device requiring information from the blood pressure monitor 101. The wireless communication protocol for communicating with a nearby mobile phone or a computer is preferably Bluetooth Low Energy.

Optionally, the blood pressure monitor 101 comprises a haptic feedback component 311 for issuing an alarm to the subject wearing it. Alternatively, the alarm may be replaced by or may include an audio alarm such as a small siren, or a visual alarm such as a blinking LED.

A replaceable and rechargeable battery 307 is provided to supply power to all the components in the blood pressure monitor 101. The battery 307 is preferably a rechargeable and replaceable one, as it allows the subject to swap the battery 307 quickly at any time, such that there is no need to wait for the battery 307 to charge up. This provides the advantage that the subject may benefit from almost seamless, continual monitoring of his blood pressure over long periods of use.

Optionally, the blood pressure monitor 101 also comprises a skin impedance sensor 315 (not illustrated in Figure 1) positioned on the underside of the blood pressure monitor 101, adjacent the PPG sensor 309. A skin impedance sensor 315 measures impedance or conductance of skin surface. Impedance of skin is different from that of air. Therefore, if the skin impedance sensor 315 is in contact with the skin of the subject, certain impedance should be measurable. If there is a small gap between the subject's skin and the PPG sensor 309, there will also be a small gap between the skin impedance sensor 315 and the skin, such that the skin impedance sensor 315 is unable to detect impedance typical of skin but detect impedance somewhat typical of air. In this way, the skin impedance sensor 315 is useable to determine whether the PPG sensor 309 has been placed in sufficient contact with the skin in order for the PPG sensor 309 to read heart beat properly without any gap for intruding ambient light affecting the reading of the PPG sensor 309.

Preferably, the blood pressure monitor 101 is able to alert the subject if the PPG sensor 309 is not placed tightly enough against the skin, such as by issuing a series of haptic signals in a specific rhythm.

In addition, the impedance sensor is also used to monitor whether the subject's skin is too dry. If the skin is too dry, the ECG signal will be too weak and there will not be a sufficiently accurate ECG measurement.

Optionally, the blood pressure monitor 101 comprises an accelerometer 313 which may be used to monitor the movements of the subject, such as the subject's step count. This information can be used to further interpret the state of the subject's health and blood pressure.

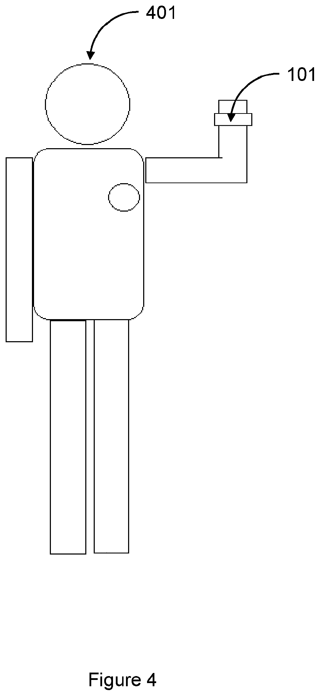

Figure 4 further illustrates how a subject 401 may wear the blood pressure monitor 101 on his wrist as a wrist band. In other embodiments, the blood pressure monitor 101 may be configured to be worn on other parts of the body, such as on the ankle, around the neck or around the head or temples (both not illustrated).

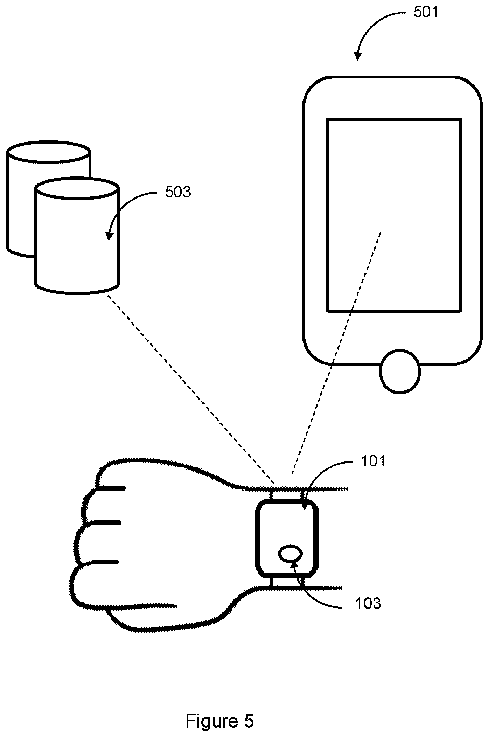

Figure 5 illustrates that the blood pressure monitor 101 is capable of wireless communication directly with a mobile phone 501 and/or a server 503. Preferably, a mobile application is installed in the subject's mobile phone 501 to collect data from the blood pressure monitor 101, and to display the data and an analytical report of his blood pressure (and any other information obtained by the blood pressure monitor 101 such as the subject's step count), as well as forwarding the data to the server 503 for storage. If an alarm of possibly risky blood pressure is raised, be the blood pressure too high or too low, the mobile application in the mobile phone 501 is able to display an alert message and important information for first aid or nearest medical facility.

Preferably, the blood pressure monitor 101 is able to issue an alarm over the Internet or telecommunication network to a specific caregiver or an emergency service provider. Therefore, in another embodiment, the blood pressure monitor 101 is part of a smart watch (not illustrated) with its own Internet communication functions and user interactive abilities, bypassing the need for an application in a smart phone.

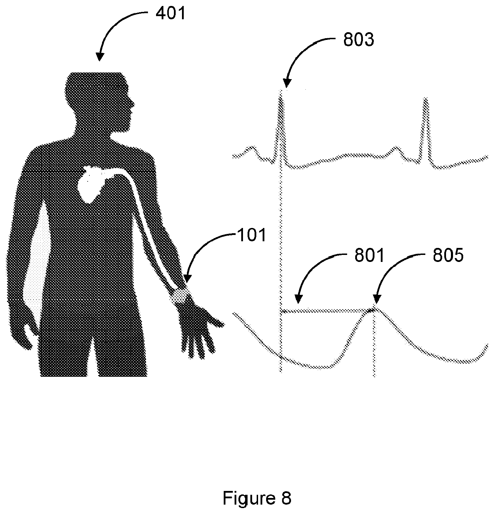

The blood pressure monitor 101 measures blood pressure of the subject 401 by observing pulse transit time (PTT). Figure 8 illustrates the pulse transit time 801 between the peak of the signal 803 read by the ECG sensor and the peak of the signal 805 read by the PPG sensor, both the ECG and the PPG readings being of the same heart beat.

The ECG sensor signal is virtually read in exact time of the subject's heart beat, since ECG is read from the heart's electrical impulses. The PPG sensor, however, monitors the flow of blood the in the wrist on which the embodiment is worn. Hence, the time difference between the peak of the ECG sensor and the peak of the PPG sensor indicates the time taken by a pulse of blood to transit from the heart to the limb. The transit time of the pulse can be used to determine blood pressure using the Moens-Korteweg (M-K) Equation. The equation is shown below.

$$SBP = MBP_0 + \frac{2}{3} \cdot PP_0 \cdot \left(\frac{PTT_0}{PTT}\right)^2 \qquad \text{-------(1)}$$

$$DBP = MBP_0 - \frac{1}{3} \cdot PP_0 \cdot \left(\frac{PTT_0}{PTT}\right)^2 \qquad \text{-------(2)}$$

SBP = Systolic Blood Pressure
DBP = Diastolic Blood Pressure
MBP = Mean Blood Pressure
PP = Pulse Pressure
PTT = Pulse Transit Time
Subscript 0 denotes calibration point

There are actually two sub-equations making up the whole Moens-Korteweg Equation. Equation (1) measure the Systolic Blood Pressure, while Equation (2) is the counterpart of Equation (1), and measures the DBP.

Using a light and electrical impulses to determine the blood pressure of a subject 401 means that the blood pressure monitor 101 does not need to rely on a pressurizing cuff to monitor blood pressure, and therefore a cuff-less and non-invasive blood pressure monitor is possible. All the subject needs to do is to wear the blood pressure monitor 101 on one wrist, and place a finger of his other hand onto the ECG electrode 105 on the top side of the blood pressure monitor 101, and wait for one or more of his heart beats to be observed for their ECG and PPG activities to be read and converted into blood pressure.

Before the blood pressure monitor 101 can be used to interpret pulse transit time into blood pressure, the blood pressure monitor 101 has first to be calibrated to do so. To provide a one point calibration, $MBP_0$ of the specific subject 401 is obtained by reading his blood pressure using a sphygmomanometer while measuring the pulse transit time concurrently. The reading of the pulse transit time provides $PTT_0$. Hence, this obtains a known blood pressure and a known pulse transit time against which future readings of pulse transit time of the same subject may be referenced. Typically, the Omeron JPN1 electronics sphygmomanometer is used to measure the blood pressure of the subject 401 to benchmark the PTT to blood pressure measurements.

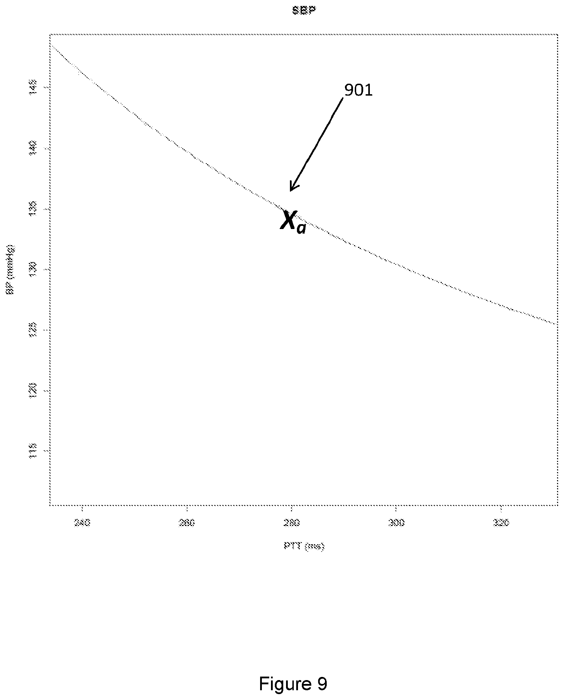

Once $MBP_0$ and $PTT_0$ are obtained, Equation (1) and Equation (2) are useable to provide a one-point calibration for SBP and DBP, respectively. As the one $MBP_0$ to one $PTT_0$ is used to establish their relationship, the calibration is a one point calibration. Subsequently, by observing the pulse transit time at any instant, the subject's blood pressure can be deduced using Equation (1) and/or Equation (2). Figure 9 is a graph of Equation (1) for SBP (systolic blood pressure) after $MBP_0$ and $PTT_0$ has been obtained.

In practice, interpretation of PTT into SBP and DBP using one point calibrations tends to give readings of blood pressure that deviate to either side of the calibrations, especially if the calibrations were made a while ago. However, this drift of calibration is not systematic but is random and cannot be predicted by formula or mathematical modelling.

The many reasons for calibration drift include the subject's normal, daily physiological variation, such as changes in arterial stiffness through a day or even through a week, and so on. The main contributing factor of physiological variation is change in sympathetic activity of the autonomous nervous system, which regulates breathing rate, heart rate, blood pressure, etc.

There is no formula for considering an infinite number of physiological parameters to predict all causes of calibration drift. In other words, there is an operational limit to the range of PTT values that the M-K equation is able to model. More specifically, if the measured PTT value is too far away from the $PTT_0$ value, i.e. the original calibration point, the ability of the M-K equation to deduce blood pressure value from reading the subject's PTT is diminished.

Therefore, to address calibration drift, a plurality of single-point calibrations is used in the preferred embodiment.

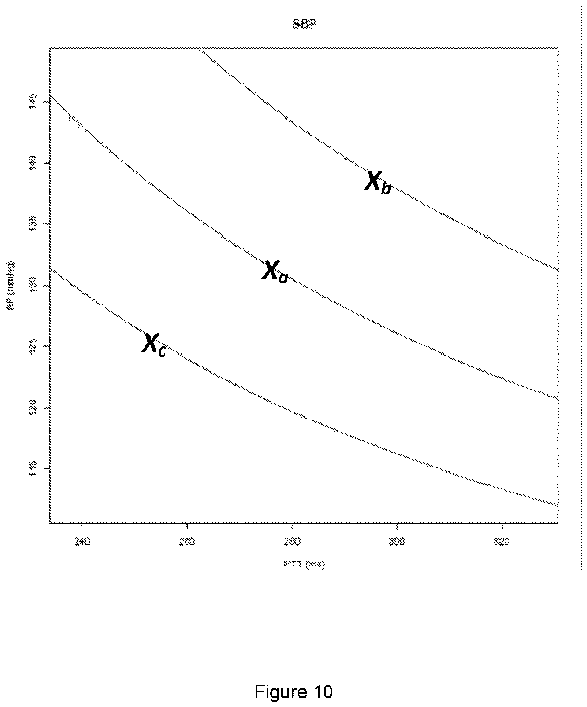

Figure 10 shows such a plurality of one point calibrations $X_a$, $X_b$ and $X_c$, each obtained using the same M-K equation (1) for SBP. When the blood pressure monitor 101 is used to monitor the subject's blood pressure, interpretation of PTT has to be made after selecting the most suitable of the calibrations, $X_a$, $X_b$ or $X_c$.

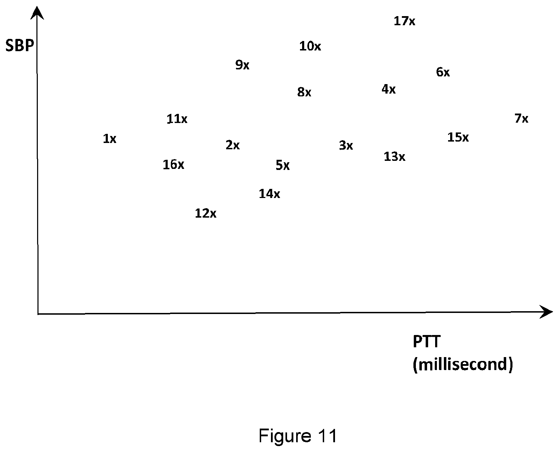

Figure 11 to Figure 18 is a series of schematic diagrams explaining how the blood pressure monitor 101 may be calibrated using a plurality of one point calibrations and then used to calculate SBP by selecting the most suitable one of the one point calibrations. As can be seen in Figure 11 to Figure 18, the y-axis represents SBP, while the x-axis represents PTT in milliseconds.

In Figure 11, a plurality of PTTs is obtained for calibration from the subject 401 wearing the blood pressure monitor 101 at the same time as measuring his blood pressure by a sphygmomanometer. Preferably, each pair of PTT and corresponding blood pressure is obtained on different days in order that observations may be taken over as wide a range of different physiological conditions of the subject as possible, and are each represented by the small letter "x". There are seventeen pairs of PPT point and blood pressure reading in Figure 11, hence points 1 x to 17x.

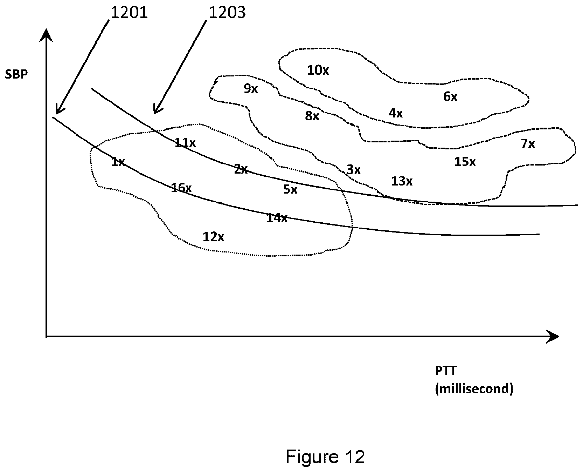

The points are not used directly for calibration. Each of points 1x to 17x is applied as $MBP_0$ and $PTT_0$ of Equation (1) to act as an interim calibration. Figure 12 shows the graph of Equation (1) applied to point 1x, labelled 1201. It can be seen that points 16x and 14x fits into Equation (1) with the values of 1x being the $MBP_0$ and $PTT_0$. Hence, points 1x, 14x and 16x are grouped together. Point 12x does not fall exactly onto the graph of point 1x but it is statistically near enough. Hence, point 12x is also grouped into the same group as 1x, 14x and 16x. Similarly, point 2x is also statistically near enough to point 1x, and is grouped along into the same group of points 1x, 14x, 16x and 12x.

In the current embodiment, 'near' is determined by difference in measured SBP. The distance in y-axis between point 1x, 14x, 16x, and 12x, to interim calibration graph 1201 has to be within a certain limit, such as 8 mmHg, which is the accuracy requirement of USA Food and Drug Administration (FDA).

Subsequently, taking point 2x as the next interim calibration point, and applying the values of point 2x as $MBP_0$ and $PTT_0$ into Equation (1), another interim calibration graph 1203 is obtained which transacts through point 2x. It can be seen that points 5x and 11x falls near enough to the graph of point 2x. Hence, points 5x, 11x are grouped along with points 2x, 1x, 16x, 14x and 12x.

The process is iterated point by point, from points 1x all through to 17x. Eventually, a clear separation of the groups will be observed, wherein all the points are grouped together into groups which are most predictive of each other when each point is used as $MBP_0$ and $PTT_0$ of Equation (1). The graph in Figure 12 shows three groups neatly identified and separated in this way.

For clarity, it should be noted that if there is a calibration point that can fall into 2 groups, the point is only assigned to the group with the closest line to the point. Optionally, if there is a calibration point that can fall into 2 groups equally and are close to two different graphs equally, the point may be eliminated from consideration. However, this should very rarely occur.

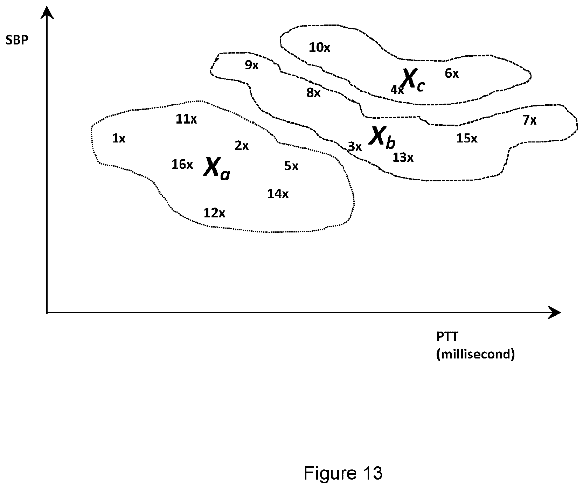

Once the groups are identified, all the points in each group are averaged, providing the 3 big "X"s seen in Figure 13. $X_a$, $X_b$ and $X_c$ are the average of all the respective points in each group.

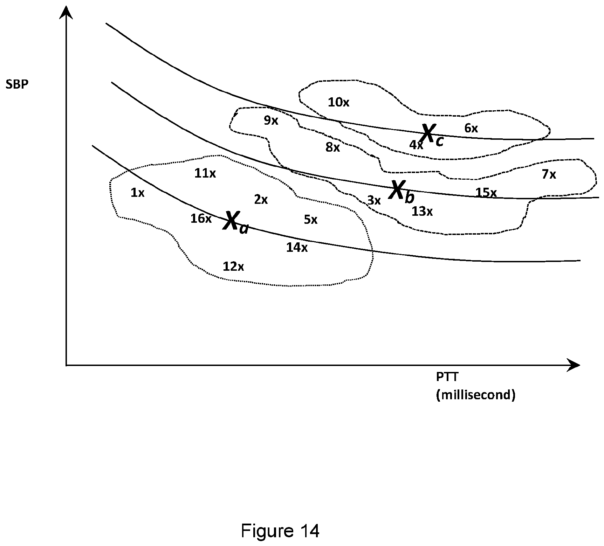

It is the big "X"s which are used to provide calibrations for determining the blood pressure of the subject 401. Hence, 3 graphs can be seen in Figure 14, each of the graph provided by feeding the respective points $X_a$, $X_b$ and $X_c$ into Equation (1). In other words, the graphs of $X_a$, $X_b$ and $X_c$ are also one point calibrations. These three graphs are used whenever the embodiment is worn and a PTT of the subject 401 is read.

The number of graphs in Figure 14 is only for illustrative purpose. In reality, there may be only two groups of blood pressure readings or there may be more than three groups, as may be discovered for any subject 401.

As the graphs of $X_a$, $X_b$ and $X_c$ are obtained based on observing the subject's blood pressure and respective PTT on different days and times, most normal physiological variation of the subject is likely to have been included in the observations without any need to know exactly what these physiological variations are. Hence, $X_a$, $X_b$ and $X_c$ are called empirical calibrations, versus one which is based strictly on a formula such as Equation (1) and which is called a theoretical calibration herein. In other words, $X_a$, $X_b$ and $X_c$ are called empirical calibrations because the calibration has taken into account physiological variation by making empirical observations.

Each of the graphs of $X_a$, $X_b$ and $X_c$ is actually a drift of the theoretical calibration. However, while calibration drift may occur randomly, it does not occur without reason. There must be a cause for the drift which is based on a change in the physiological condition of the subject. While the cause is not known, being difficult to identify from the many possible physiological variations that may happen in the subject's body, it is believed that the cause is recurrent and with certain limits. Most human bodies tend to change normally under different situations or circumstances but within such physiological limits or thresholds. For example, the subject waking up from a deep sleep tends to be less tense than the same subject who has just watched a horror movie, but these changes all occur within physiological limits which may be qualified by empirical observations.

Some physiological changes, while different from each other, may cause the same drift in the theoretical calibration. All forms of physical tension caused by emotion, exercise and lack of sleep may manifest into the same hardening of muscles or blood vessels, for example. The drift caused to the theoretical calibration will likely be similar despite emotion, exercise and lack of sleep being completely different matters. Hence, it is not important to pinpoint exactly the physiological change in the subject's body causing a calibration drift. By making observations of the drift of the theoretical calibration over a period of time, overall observations of recurrent drifts can be determined and accounted for.

The improvement of blood pressure measurement accuracy of the embodiment lies finally in the selection of which of the three empirical calibration graphs in Figure 14 to use for every PTT read by the blood pressure monitor 101. That is, the best fit of $X_a$, $X_b$ and $X_c$ has to be determined for every PTT afresh.

The best fit of $X_a$, $X_b$ and $X_c$ has to be selected for each PTT, instead of applying either one of $X_a$, $X_b$ and $X_c$ to all PTTs within a period of time, as this gives the most accurate result for each instance of heart beat.

In practice, each group of interim calibration points (1x to 17x) comprises of points altogether spanning a maximum range of 8 mmHg, so as to give a resolution of 8mm Hg between the plurality of resultant empirical calibrations.

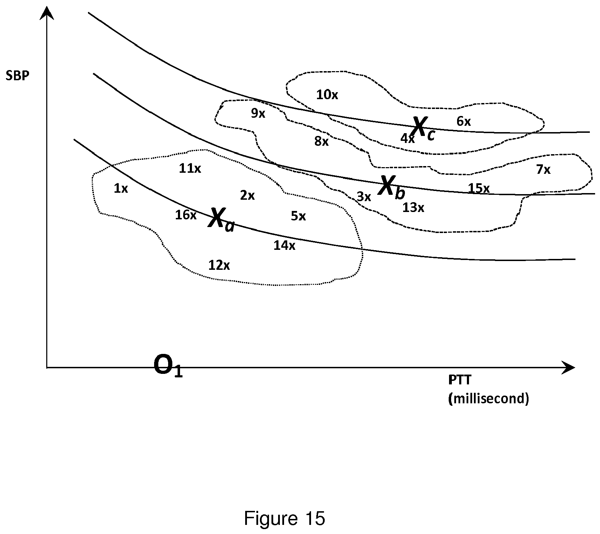

Figure 15 shows a current heart beat's PPT reading, $O_1$, taken after the calibrations have been determined. As explained before, $O_1$ is obtained from the time difference between the ECG and PPG pulses of the same heart beat. In order to select which of the empirical calibrations, i.e. $X_a$, $X_b$ and $X_c$, should be used to interpret $O_1$, each of the interim calibration points 1x to 17x are considered for its proximity to $O_1$ along the PTT axis.

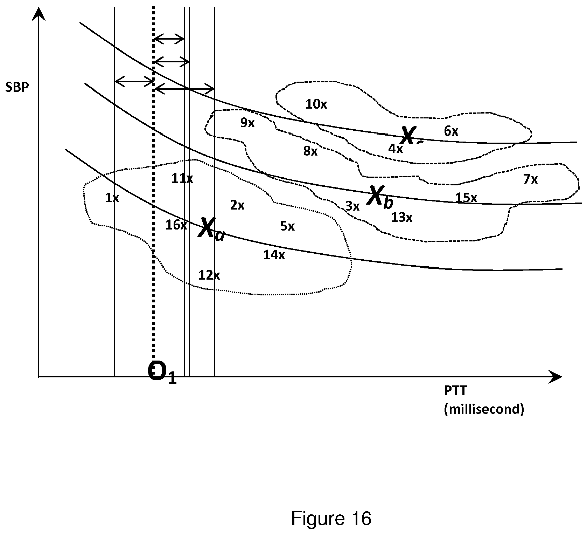

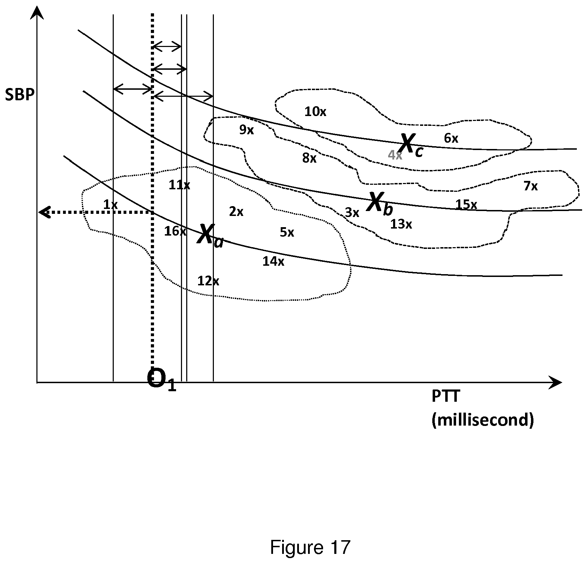

Figure 16 shows that the nearest interim calibration point is 16x. Hence, the group to which 16x belongs is used for interpreting the blood pressure represented by $O_1$, i.e. the group of $X_a$. Hence, Figure 17 shows a vertical line from $O_1$ is drawn up to the empirical calibration graph of $X_a$, and then projected horizontally to obtain the blood pressure represented by point $O_1$.

Of course, Figure 17 is only a graphical description of how to apply Equation (1). A non-graphical, but computed approach is well within the contemplation of this embodiment.

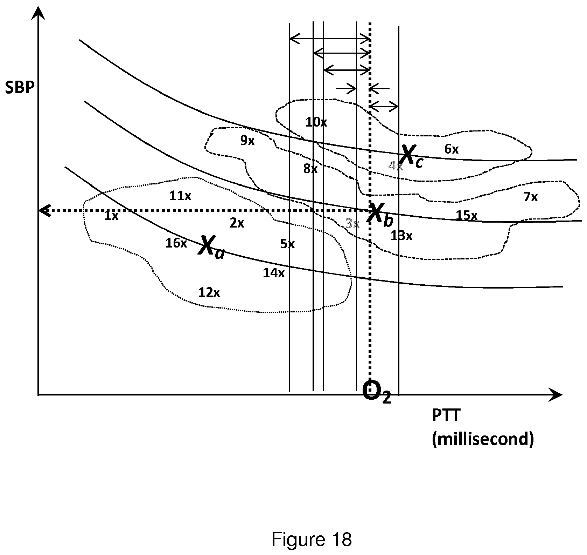

Figure 18 shows another PTT reading $O_2$ obtained by the blood pressure monitor 101. $O_2$ is nearest to the interim calibration point 3x. The group of 3x is that of empirical calibration point $X_b$. Hence, a vertical line is drawn up from $O_2$ to reach $X_b$, and then projected horizontally to read the blood pressure.

Therefore, the nearest blood pressure readings obtained during interim calibration, i.e. small x points, is selected to identify the empirical calibrations, $X_a$, $X_b$ or $X_c$, to use.

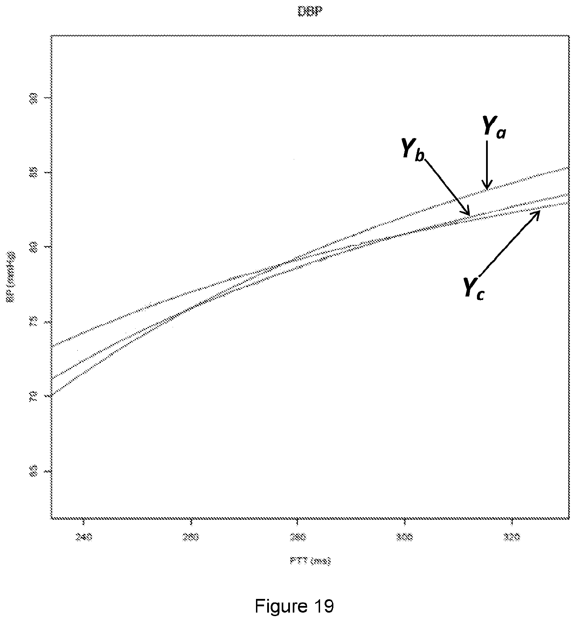

Figure 19 shows three empirical calibration graphs $Y_a$, $Y_b$, and $Y_c$, obtained in the same way as described for $X_a$, $X_b$ and $X_c$ but using Equation (2) for DBP (diastolic blood pressure) instead of Equation (1) for SBP. The same calibration steps are used with Equation (2) as described for Equation (1) in Figure 11 to Figure 18.

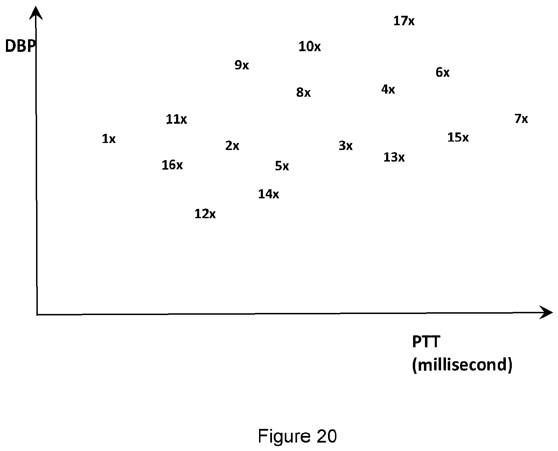

Figure 20 to Figure 23 are a series of schematic diagrams explaining how the blood pressure monitor 101 may be calibrated and then used to calculate DBP. As shown in Figure 20 to Figure 23, the y-axis represents DBP while the x-axis represents PTT in milliseconds.

In Figure 20, a plurality of PTTs is obtained for calibration from the subject 401 wearing the blood pressure monitor 101 at the same time as measuring his blood pressure by a sphygmomanometer. Preferably, each PTT and corresponding blood pressure is preferably obtained on different days in order to take readings on as varied a physiological condition of the subject as possible over time, and are each represented by the small letter "x". There are seventeen of such PPT points and blood pressure readings in Figure 20, hence points 1x to 17x.

The points are not used directly as calibration. Each of the points 1x to 17x and its respective blood pressure reading taken from the sphygmomanometer are applied as $MBP_0$ and $PTT_0$ of Equation (2) to act as an interim calibration.

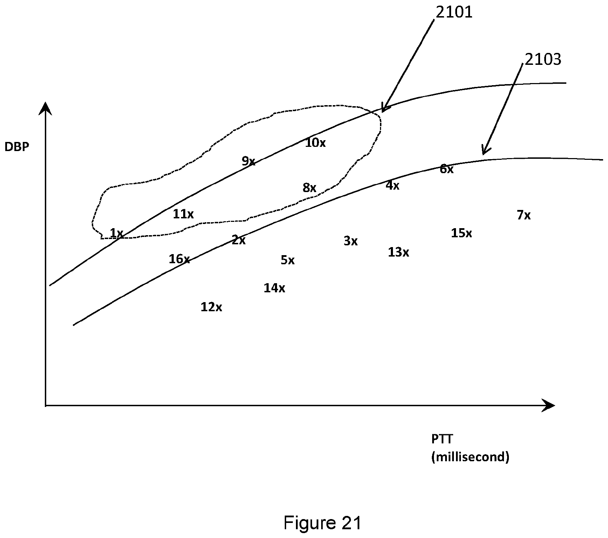

Figure 21 shows the graph of Equation (2) applied to interim calibration point 1x, labelled 2101. It can be seen that interim calibration points 8x, 9x, 11x and 10x fit into Equation (2) with the values of 1x being the $MBP_0$ and $PTT_0$. Hence, interim calibration points 1x, 8x, 9x, 10x, and 11x are grouped together. Point 8x does not fall exactly onto the graph of point 1x but it is statistically near enough. Hence, point 8x is also grouped into the same group as 1x.

Similarly, interim calibration points 16x, 2x, 12x, 14x, 5x, 3x, 4x and 6x are grouped together, and interim calibration points 13x 5x and 7x are grouped together.

Subsequently, taking point 2x in turn as an interim calibration point, and applying the values of point 8x as $MBP_0$ and $PTT_0$ into Equation (2), an interim calibration graph 2103 is produced transacting through point 8x. It can be seen that points 16x, 4x and 6x falls near enough to the graph of point 2x. Hence, points 8x, 1x, 11x are grouped together.

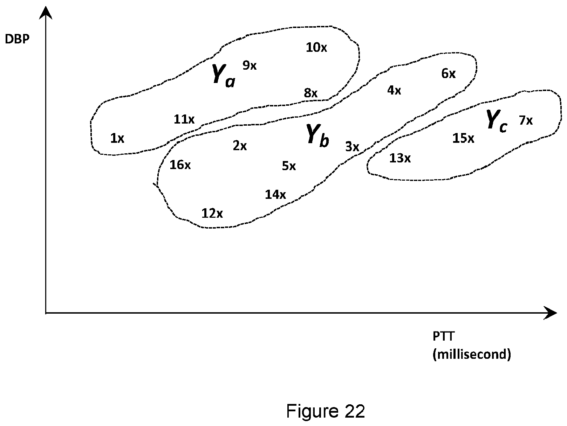

The process is repeated or iterated point by point, from points 1x all through to 17x. Eventually, all the points are grouped together into groups which are most predictive of each other when each point is applied as the interim calibration point $MBP_0$ and $PTT_0$ of Equation (2). The graph in Figure 22 shows three groups neatly identified and separated in this way.

If there is a calibration point that can fall into 2 groups, the point is assigned to the group with the closest line to the point. Alternatively, if there is a calibration point that can fall into 2 groups equally and are close to two different graphs equally, the point is eliminated from consideration. However, this should very rarely occur.

Once the groups are identified, all the points in the group are averaged, providing the 3 big "Y"s seen in Figure 22. $Y_a$, $Y_b$ and $Y_c$ are each an average of all the points in each respective group. It is the big "Y"s which are used to provide calibrations which actually used for determining the blood pressure of the subject 401.

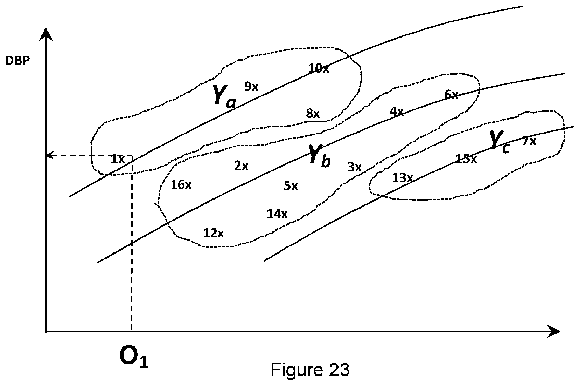

Hence, three graphs can be seen in Figure 23, each one based on an averaged calibration point, $Y_a$, $Y_b$ or $Y_c$. These three graphs are used whenever the embodiment is worn and a PTT of the subject 401 is read, to interpret the PTT into DBP.

$Y_a$, $Y_b$ and $Y_c$ are empirical calibrations based on empirically observing drift of the theoretical calibration of Equation (2), advantageously without needing to know exactly which physiological factors caused the drift.

As mentioned, the number of graphs in Figure 23 is only for illustrative purpose. In reality, there may be only two groups of blood pressure readings or there may be more than three groups, as may be discovered for the subject 401.

The improvement of measurement accuracy of the embodiment lies in the selection of which of the three calibration graphs in Figure 23 to use for every PTT read by the blood pressure monitor 101.

Hence, Figure 23 shows a PPT reading, $O_1$, taken after the calibrations have been determined. In order to determine which of the empirical calibrations $Y_a$, $Y_b$ and $Y_c$ should be used to interpret $O_1$, each of the interim calibration points 1x to 17x are considered for its proximity to $O_1$ along the PTT axis. Figure 23 shows that the nearest interim calibration point to $O_1$ is 1x. Hence, then the group to which 1x belongs is used for interpreting the blood pressure represented by $O_1$, i.e. the group of $Y_a$. Hence, Figure 23 shows a vertical line from $O_1$ is drawn up to the graph of $Y_a$, and then projected horizontally to obtain the blood pressure represented by point $O_1$.

Therefore, the nearest blood pressure readings obtained during calibration, i.e. small x points, is selected to identify the calibrations, $Y_a$, $Y_b$ or $Y_c$, to use.

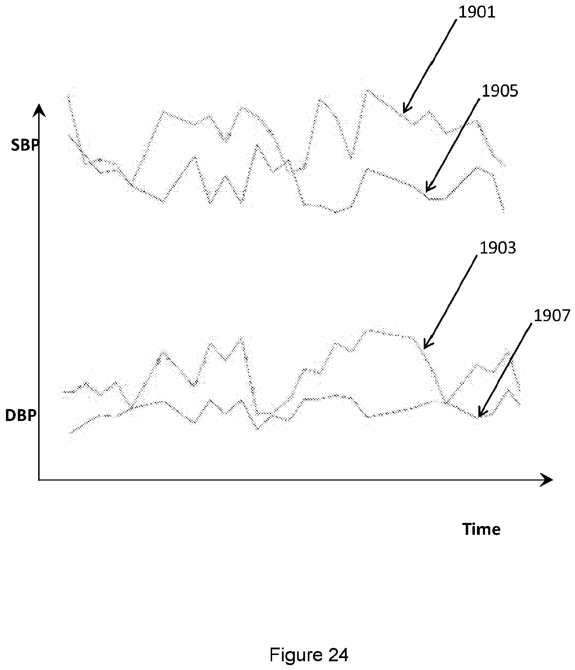

Figure 24 shows the relatively inferior accuracy of reading the SBP and DBP by using just one single-point theoretical calibration to interpret all PTT readings. The actual SBP is represented by the line 1901, and the actual DBP is represented by the line 1903. SBP detected using the subject's PTT and interpreted against one single-point calibration is represented by the lines 1905. DBP detected using the subject's PTT and interpreted against one single-point calibration is represented by the line 1907.

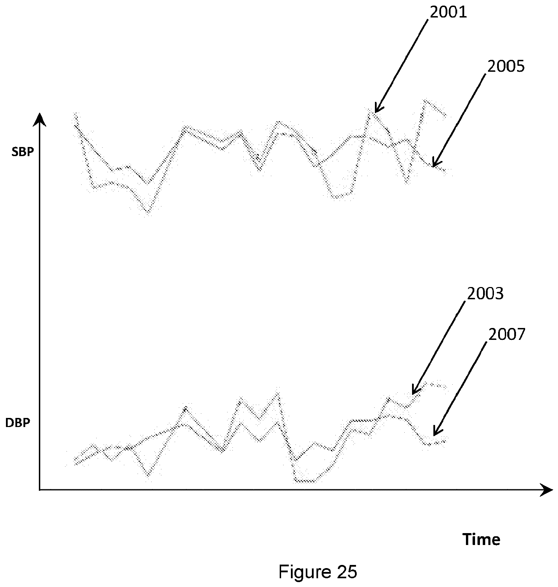

Figure 25 shows the superior accuracy of reading the SBP and DBP by using an empirical calibration dynamically selected afresh for every PTT from among a plurality of single-point empirical calibrations. The actual SBP is represented by the line 2001, and the actual DBP is represented by the line 2003. SBP detected using the subject's current PTT and interpreted against the selected empirical calibration based on Equation (1) is represented by the line 2005. DBP detected using the subject's current PTT and interpreted against the selected one point empirical calibration based on Equation (2) is represented by the line 2007.

It can be seen at a glance that between Figure 24 and Figure 25, the use of a dynamically selected empirical calibration from among a few empirical calibrations to interpret every PTT reading into blood pressure is more accurate than the use of just one theoretical calibration for all PTT readings. The actual blood pressure and the deduced blood pressure in Figure 25 are a lot closer.

Other factors can be used to improve the calibration. For example the breathing rate of the subject 401 may be included into the calibration. For example, the points 1x to 17x in Figure 11 to Figure 18 for SBP and in Figure 20 to Figure 23 for DBP may be further differentiated into points taken when the subject 401 is breathing faster, and points taken when the subject 401 is breathing slower.

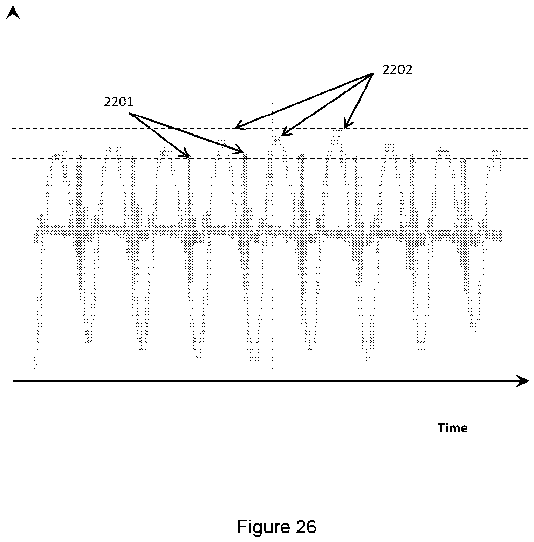

The state of breath of the subject 401 is easily observed from the ECG sensor or PPG sensor signal, as the breath of the subject 401 varies the fluctuation of the peaks, imparting a relatively low frequency signal component. This may be seen in Figure 26. Figure 26 shows an ECG sensor chart 2201 of a subject 401, superimposed onto his PPG sensor chart 2202. The higher ones of the upper peaks of the ECG sensor and PPG sensor charts correspond to the inhalation of the subject 401 while the lower ones of the upper peaks of the ECG sensor and PPG sensor charts correspond to the exhalation, i.e. the fluctuations between the two dashed lines in Figure 26 correlates to the subject's breathing.

Besides the state of the subject's breath, other factors can be used to provide different calibrations for the embodiment more finely. For example the time of peak to peak interval between every two successive heart beats may be included into the calibration. For example, there may be a plurality of calibrations of pulse transit time to blood pressure as described, but each further categorised according to a predetermined peak to peak period such as 0.70 to 0.79 seconds, 0.80 to 0.89 seconds, 0.90 to 0.99 seconds and so on. This further discrimination between different calibrations allows a finer selection of the most suitable calibration to be used to interpret the pulse transit time into blood pressure.

The peak to peak interval can be between two peaks of the ECG sensor or between two peaks of the PPG sensor; either one of the ECG sensor or PPG sensor can be used.

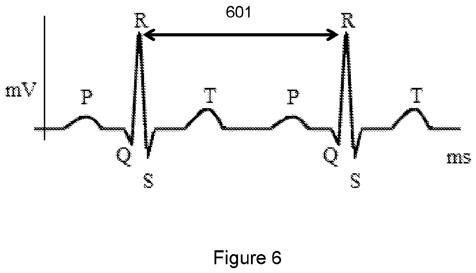

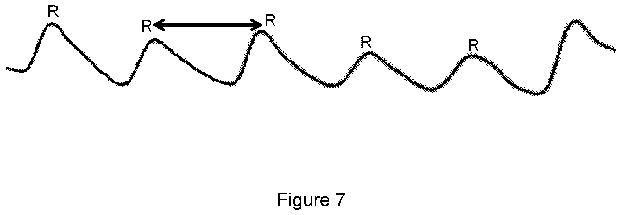

Figure 6 shows two successive pulses sampled in an ECG sensor. Every ECG sensor pulse has the peaks and troughs labelled PQRST, where P is the point where there is atrial contraction (top heart chamber), R is the point where there is ventricular contraction (bottom heart chamber) and T is the point where there is relaxation. The peak R is the largest peak in each pulse, and is the easiest point by which the interval between two pulses is measured. Therefore, the interval between two pulses is known as the RR interval 601. Sometimes, it is known as the NN interval, meaning "normal to normal" interval. On the other hand, Figure 7 shows a chart of heart beat obtained by the PPG sensor 309 in the blood pressure monitor 101. The peak to peak period is also labelled as '601' in Figure 7.

In other embodiments, the described method omits the step of grouping the calibration points and averaging them. Each of the plurality of PTTs obtained for calibration from the subject 401 is simply used to provide an empirical calibration. The interim calibration points 1x to 17x in Figure 11 and Figure 19 are used directly as empirical calibrations without need to calculate for $X_a$, $X_b$ and $X_c$ or $Y_a$, $Y_b$ and $Y_c$. The best fit of the empirical calibrations provided by points 1x to 17x can be selected based on the proximity of the PTT of the current heartbeat to 1x to 17x . In this case, the method of relating pulse transit time to blood pressure may comprise the steps of: obtaining the pulse transit time of a current heart beat travelling from the heart to a peripheral of a subject; selecting an empirical calibration out of plurality empirical calibrations for interpreting the pulse transit time into blood pressure; wherein the empirical calibration is selected if the pulse transit time used to provide the empirical calibration is the nearest of all the pulse transit times used to provide the plurality of empirical calibrations to the pulse transit time of the current heart beat.

Furthermore, besides the described formula in Equation (1) and Equation (2), other formulae can be used as long as the relation of SBP can be correlated to the PTT and the DBP can be correlated to the PTT. The theoretical model of different formulae may be more accurate or less, but they can all be augmented or supplemented by making empirical observations to calibration drift. Such other formulae are within the contemplation of this invention.

Therefore, embodiments have been described which include a blood pressure monitor 101 that relies on the pulse transit time (PTT) of a heart beat travelling from the heart to a part of the subject's body such as his limb. The calibration of the blood pressure monitor 101 is partly based on a theoretical calibration. However, to address calibration drifts which cause inaccuracy, the theoretical calibration is augmented by empirical observations. That is, variations from the theoretical calibration are obtained as a set of calibrations based on empirical observations. The blood pressure monitor 101 is then made more accurate by selecting the best fit of the set of empirical calibrations for interpreting any instant PTT into blood pressure.

In other words, a device 101 and a method which monitors the pulse transit time of a heart beat has been disclosed, i.e. the time taken for a heart impulse to travel from the heart to a limb of a subject 401. The time taken to travel is calibrated to blood pressure of the subject 401 by a pre-determined formula (Equation (1) and /or Equation (2)). However, the calibration is augmented by empirical observations, obtaining several optional calibrations. The most suitable of the calibrations is selected to interpret each pulse transit time reading based on proximity of the pulse transit time. Hence, it is possible that every pulse transit time reading may use a different calibration. The method diminishes the effect of physiological change in the subject 401 which causes drift in a solely formula based calibration.

While there has been described in the foregoing description preferred embodiments of the present invention, it will be understood by those skilled in the technology concerned that many variations or modifications in details of design, construction or operation may be made without departing from the scope of the present invention as claimed.

**Claims**

1. A method of relating pulse transit time to blood pressure comprising:

   reading the pulse transit time of a current heart beat travelling from the heart to a peripheral of a subject, and selecting an empirical calibration out of a plurality of empirical calibrations for interpreting the pulse transit time into blood pressure; each of the plurality of empirical calibrations having been obtained by earlier steps of:

   averaging a plurality of observed blood pressure and respectively observed pulse transit times to obtain an averaged observed pulse transit time and an averaged observed blood pressure; and
   applying the averaged observed pulse transit time and averaged observed blood pressure to either or both of formulae (1) and (2):

$$SBP = MBP_0 + \frac{2}{3} \cdot PP_0 \cdot \left(\frac{PTT_0}{PTT}\right)^2 \qquad \text{-------(1)}$$

$$DBP = MBP_0 - \frac{1}{3} \cdot PP_0 \cdot \left(\frac{PTT_0}{PTT}\right)^2 \qquad \text{-------(2)}$$

SBP = Systolic Blood Pressure
DBP = Diastolic Blood Pressure
MBP=Mean Blood Pressure
PP=Pulse Pressure
PTT=Pulse Transit Time
Subscript 0 denotes calibration point;

wherein the empirical calibration is selected out of the plurality of empirical calibrations based on a closest of the observed pulse transit times making up the several predetermined calibrations to the pulse transit time of the current heart beat.

2. .A method of relating pulse transit time to blood pressure as claimed in claim 1, wherein the empirical calibrations are one point calibrations.

3. .A method of relating pulse transit time to blood pressure as claimed in claim 1, wherein the peripheral is a limb of the subject.

4. A method of relating pulse transit time to blood pressure as claimed in claim 3 wherein the peripheral is the wrist of the subject.

5. A method of relating pulse transit time to blood pressure as claimed in claim 1, wherein
the calibrations are **characterized by** the state of the subject's breath; the state of the breath being the rate of inhalation or exhalation of the subject when a pulse transit time is measured; and wherein
the calibration is selected out of several predetermined single-point calibrations based on the closest of observed pulse transit times making up the several predetermined calibrations to the read pulse transit time of the current heart beat as well as based on the same state of breath.

6. A method of relating pulse transit time to blood pressure as claimed in claim 1, wherein:

the calibrations are **characterized by** the peak to peak period between a pulse immediately preceding the present pulse; and wherein
the calibration is selected out of the several predetermined calibrations based on a closest of observed pulse transit times making up the several predetermined calibrations to the read pulse transit time of the current heart beat as well as based on a similar peak to peak period.

7. A method of relating pulse transit time to blood pressure comprising:

obtaining a pulse transit time of a current heart beat travelling from a heart to a peripheral of a subject, and selecting an empirical calibration out of a plurality of empirical calibrations for interpreting the pulse transit time into blood pressure; wherein
the empirical calibration is selected if the pulse transit time used to provide the empirical calibration is a nearest of a plurality of pulse transit times used to provide the plurality of empirical calibrations to the pulse transit time of the current heart beat.

8. A blood pressure monitor for monitoring the blood pressure of a subject, comprising:

an electrocardiogram sensor and a photoplethysmocharty sensor configured for observing pulse transit time of a beat from a heart to a peripheral of the subject;
the blood pressure monitor configured to apply one of a plurality of calibrations to interpret pulse transit time into blood pressure; wherein
the plurality of calibrations are variations of a same formula-based calibration, and
the variations having been observed in the subject.

9. A blood pressure monitor for monitoring the blood pressure of a subject as claimed in claim 8, wherein the formula is either or both of formulae (1) and (2):

$$SBP = MBP_0 + \frac{2}{3} \cdot PP_0 \cdot \left(\frac{PTT_0}{PTT}\right)^2 \qquad \text{-------(1)}$$

$$DBP = MBP_0 - \frac{1}{3} \cdot PP_0 \cdot \left(\frac{PTT_0}{PTT}\right)^2 \qquad \text{-------(2)}$$

SBP = Systolic Blood Pressure
DBP = Diastolic Blood Pressure
MBP=Mean Blood Pressure
PP=Pulse Pressure
PTT=Pulse Transit Time
Subscript 0 denotes calibration point.

10. A blood pressure monitor for monitoring the blood pressure of a subject as claimed in claim 9, wherein

the variations having been observed from
a plurality of observed blood pressures and respectively observed pulse transit times in the subject.

11. A blood pressure monitor for monitoring the blood pressure of a subject as claimed in claim 10, wherein

the plurality of calibrations providing the variations are groups of averaged pluralities of observed blood pressures and respectively observed pulse transit times in the subject.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

Figure 21

Figure 22

Figure 23

Figure 24

Figure 25

Figure 26

Europäisches
Patentamt

European
Patent Office

Office européen
des brevets

# EUROPEAN SEARCH REPORT

Application Number

EP 18 17 8332

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/028011 A1 (HUAWEI TECH CO LTD [CN]) 23 February 2017 (2017-02-23) | 7,8 | INV. A61B5/021 |
| A | * the whole document * | 1-6,9-11 | |
| X,P | US 2018/078156 A1 (CHEN WENJUAN [CN] ET AL) 22 March 2018 (2018-03-22) * paragraph [0006] - paragraph [0053] * * paragraphs [0130], [0131] * * figure 5 * | 7,8 | |
| A | US 2016/302677 A1 (HE DAVID DA [US]) 20 October 2016 (2016-10-20) * paragraph [0003] - paragraph [0008] * * paragraph [0121] - paragraph [0123] * * figure 13 * | 1,7,8 | |
| A | WO 2016/155138 A1 (VITA-COURSE TECH CO LTD [CN]) 6 October 2016 (2016-10-06) * paragraph [0003] - paragraph [0013] * * figures 1-15 * | 1,7,8 | |

**TECHNICAL FIELDS SEARCHED (IPC)**

A61B

The present search report has been drawn up for all claims

| Place of search | Date of completion of the search | Examiner |
|---|---|---|
| Berlin | 18 October 2018 | Abraham, Volkhard |

EPO FORM 1503 03.82 (P04C01)

## ANNEX TO THE EUROPEAN SEARCH REPORT
## ON EUROPEAN PATENT APPLICATION NO.

EP 18 17 8332

This annex lists the patent family members relating to the patent documents cited in the above-mentioned European search report.
The members are as contained in the European Patent Office EDP file on
The European Patent Office is in no way liable for these particulars which are merely given for the purpose of information.

18-10-2018

| Patent document cited in search report | | Publication date | Patent family member(s) | | Publication date |
|---|---|---|---|---|---|
| WO 2017028011 | A1 | 23-02-2017 | CN<br>US<br>WO | 107072555 A<br>2018078156 A1<br>2017028011 A1 | 18-08-2017<br>22-03-2018<br>23-02-2017 |
| US 2018078156 | A1 | 22-03-2018 | CN<br>US<br>WO | 107072555 A<br>2018078156 A1<br>2017028011 A1 | 18-08-2017<br>22-03-2018<br>23-02-2017 |
| US 2016302677 | A1 | 20-10-2016 | NONE | | |
| WO 2016155138 | A1 | 06-10-2016 | CN<br>CN<br>CN<br>US<br>US<br>US<br>WO<br>WO<br>WO | 107847158 A<br>108348172 A<br>204515353 U<br>2018085011 A1<br>2018085012 A1<br>2018192900 A1<br>2016155138 A1<br>2016155348 A1<br>2017167013 A1 | 27-03-2018<br>31-07-2018<br>29-07-2015<br>29-03-2018<br>29-03-2018<br>12-07-2018<br>06-10-2016<br>06-10-2016<br>05-10-2017 |

EPO FORM P0459

For more details about this annex : see Official Journal of the European Patent Office, No. 12/82